# EUROPEAN PATENT SPECIFICATION

(11) **EP 0 693 508 B1**
(45) Date of publication and mention of the grant of the patent: **06.05.1998**
(21) Application number: 95907867.6
(22) Date of filing: 10.02.1995
(51) Int. Cl.: C08F 220/56, C08F 220/06, C08L 33/26

(54) **TEMPERATURE-SENSITIVE WATER-ABSORBING/DESORBING POLYMER COMPOSITION**
TEMPERATUREMPFINDLICHE WASSERABSORBIERENDE/-DESORBIERENDE POLYMERZUSAMMENSETZUNG
COMPOSITION POLYMERE HYDROABSORBANTE/DESORBANTE ET THERMOSENSIBLE

(30) Priority: 10.02.1994 JP 36387/94; 28.09.1994 JP 257349/94
(43) Date of publication of application: 24.01.1996
(73) Proprietor: KOHJIN CO. Ltd., Minato-ku Tokyo 105 (JP)
(72) Inventor: MURAYAMA, Teiichi, Kumamoto 866 (JP); MARUYAMA, Takashi, Kumamoto 866 (JP)
(74) Representative: Hansen, Bernd, Dr. Dipl.-Chem.
(86) International application number: PCT/JP95/00183
(87) International publication number: WO 95/21876

(56) References cited:
- JP-A- 4 139 206
- JP-A- 4 298 203
- JP-A-61 247 716

## Description

### Technical Field

The present invention concerns a novel water absorbing resin having water absorbability that changes greatly at a certain temperature near room temperature as a boundary and repeating water absorption and discharging in accordance with the temperature.

### Background Art

A water absorbing resin has been used in various application uses, for example, as sanitary materials such as menstrual articles, diapers and disposable napkins or as water retaining agent in agricultural or horticultural uses or condensation prevention in building materials. As such a water absorbing resin, hydrogels using carboxymethyl cellulose crosslinking products, starch - acrylonitrile graft copolymers, polyvinyl alcohols and polyacrylates as the raw materials have been generally known. However, such hydrogels mentioned above have only one function of starting water absorption upon contact with water.

However, it has been attempted in recent years to further add other functions. For instance, as shown in Japanese Patent Laid-Open sho 61-55180, a non-ionic hydrogel having a function of reversibly repeating water absorption and discharging depending on the change of temperature has been known. That is, the hydrogel scarcely shows water absorption if a water temperature is higher than a certain temperature and can absorb water if the temperature lowers to less than the certain temperature. However, since the hydrogel is non-ionic, it has small water absorbing amount at a low temperature and it is not practical. For increasing the water absorbing amount, it has been reported a copolymerized hydrogel of n-isopropyl acrylamide and an ionic monomer such as a sodium acrylate, for example, in USP No. 4,732,930 and J. Chem. Phys. 1987, 87, 1392.

However, although the hydrogel with the ionic monomer described above is improved for the water absorbing amount, it involves a drawback that the inherent temperature sensing point (a boundary temperature that water absorbability exits lower than the temperature but is almost lost at a higher temperature) changes greatly by merely copolymerizing the ionic monomer slightly, more specifically, a drawback that the temperature sensing point rises as the content of the ionic monomer is increased making it difficult to obtain a composition showing a temperature sensitivity near the room temperature.

Further, various methods have been proposed for improving the water absorbing characteristic of a gel having a sensitive and reversible water absorbing and discharging performance relative to the change of temperature in water. For instance, there have been known, for example, a method of mixing iron oxide in an aqueous solution of polyvinyl methyl ether, irradiating γ-rays, thereby improving porosity and heat transfer rate to improve the water absorbing rate (J. Chem. Eng. Japan, 21, 10, 1988), a method of previously mixing hydroxyl propyl cellulose upon synthesis of a gel, cleaning the gel after polymerization to remove hydroxypropyl cellulose, thereby making the gel porous to improve the water absorbing rate (J. Polym. Soc.: Part A, 30, 2121, 1992) and a method of synthesizing a gel having not homogeneous crosslinking structure by polymerization at a temperature higher than a volume transition temperature (polymer communications, 32, 322, 1991). On the other hand, various improvements for water absorbing characteristic have also been conducted on general highly absorbing resins having no water absorbing and discharging performance relative to the change of the temperature. For instance, there have been reported, for example, a method of mixing a water absorbing resin powder with hydrotalcite to improve the water absorbing rate (Japanese Patent Laid-Open Hei 2-215863), and a method of mixing a water absorbing resin powder with attapulgite to improve the water absorbing rate (Japanese Patent Laid-Open Sho 61-58657).

However, although the methods can improve the water absorbing rate, they involve problems in that the production process for the gel is extremely complicate or the water absorbing rate of the resultant gel is not sufficient.

### Disclosure of the Invention

The present inventors, et al have made earnest studies for obtaining a resin free from the foregoing drawbacks and, as a result, have found that when N-alkyl acrylamide derivative and acrylic acid or alkali metal salt of acrylic acid are copolymerized in an aqueous solution, a temperature sensitive hydrogel maintaining high water absorbing and discharging property can be obtained by introducing diacetone acrylamide and/or acrylamide, the temperature sensitive point can be adjusted within a wide range in a lower temperature region by changing the amount of diacetone acrylamide to be introduced and, therefore, the temperature sensitive point depending on the aimed temperature can be set easily, to complete the present invention.

That is, the present invention provides a temperature sensitive water absorbing and discharging polymer composition obtained by copolymerizing an aqueous solution containing N-isopropyl acrylamide and/or N,N-diethyl acrylamide, acrylic acid and/or alkali metal salt of acrylic acid and diacetone acrylamide and/or acrylamide in the presence of a crosslinking agent.

The blending amount of the N-isopropyl acrylamide and/or N,N-diethyl acrylamide is desirably from more than 30 mol%, more preferably, more than 50 mol% of the entire monomer used in view of the temperature sensitivity although it depends on the amount of other monomers used.

Further, as the acrylic acid and/or acrylate, acrylic acid and/or alkali metal salt of acrylic acid is preferred. As the alkali metal salt of acrylic acid, there can be mentioned, for example, alkali acrylate such as sodium acrylate, potassium acrylate, calcium acrylate and magnesium acrylate or ammonium acrylate, sodium acrylate being particularly preferred. The amount of the acrylic acid and/or acrylic acid salt used, although depending on the kind thereof, is from 0.5 to 40 mol%, more preferably, 1 to 12 mol% based on the total amount of the acrylamide derivative and/or N.N-diethyl acrylamide, diacetone acrylamide and/or acryl amide.

Since diacetone acrylamide and/or acrylamide is freely soluble to water, it can be used in a wide range of amount and it is desirably from 0.1 to 40 mol% of the entire amount of the monomer to be used, in view of the temperature sensitivity. If it is less than 0.1 mol%, no substantial effect can be obtained.

Further, as a method of producing a temperature sensitive water absorbing and discharging polymer composition having a crosslinked structure by polymerizing an aqueous monomer solution as described above, there can be mentioned, for example, a method of copolymerizing a divinyl type monomer, a method of copolymerizing a crosslinkable vinyl monomer and then crosslinking the same by a crosslinker or a method of crosslinking by radioactive rays, the method of copolymerizing the divinyl type monomer being more preferred since the production process is convenient.

As the divinyl type monomer used, a monomer having good copolymerizability with each of the monomers constituting the temperature sensitive polymer, capable of efficiently taking a crosslinked structure and providing uniform crosslinking distribution is preferred and there can be mentioned, for example, N,N-methylenebis(meth)acrylamide, ethylene glycoldi(meth)acrylate, diethylene glycol(meth)acrylate, polyethylene glycoldi(meth)acrylate, propylene glycoldi(meth)acrylate and glycerine tri(meth)acrylate. The amount of the divinyl monomer to be used is about 0.001 to 5 wt% and, preferably, about 0.01 to 1 wt% based on the entire monomer.

Further, the inventors of the present application have found that a temperature sensitive water absorbing and discharging composition sensitive to the change of temperature and excellent in water absorbing and discharging rate, is obtained by conducting aqueous solution polymerization in the presence of inorganic particles.

The inorganic particles to be present are optional and one or more of materials selected preferably from acidic white clay, diatomaceous earth and kaolin are preferred.

Referring to the amount of the inorganic particles used, they are added by 2 to 50 parts by weight, preferably, 5 to 20 parts by weight based on 100 parts by weight of the polymer forming ingredients. If they are added in excess of 50 parts by weight, the water absorbing amount of the resultant temperature sensitive water absorbing and discharging composition is lowered, whereas if it is less than 2 parts by weight, no sufficient improvement can be obtained for the water absorbing and discharging rate. The inorganic particles used here preferably have a particle size from 0.001 to 100 µm. If it is greater than 100 µm, dispersibility upon polymerization is worsened, wherein if it is less than 0.001 µm, there is a problem that no sufficient improvement can be expected for the water absorbing characteristic.

A process for producing the polymer composition according to the present invention is practiced by dissolving each of monomers constituting the temperature sensitive polymer and a divinyl type monomer added as required into a solvent, adding to the solution inorganic particles to be present optionally, mixing and dispersing them and then polymerizing in the presence of a catalyst. There is no particular restriction for the solvent usable herein so long as it dissolves the monomers and use of water as a solvent is preferred in view of the production efficiency and safety. Further, if the inorganic fine particles are added and mixed after polymerization or after drying and pulverization of the polymer, uniform dispersion of the organic particles is difficult or inorganic particles are lost and, accordingly, no sufficient improvement can be expected for the water absorbing rate.

As a process for producing the polymer composition according to the present invention, aqueous solution polymerization is essential but any of methods known generally in the aqueous solution polymerization can be used. A polymerization initiator used in this case is a water soluble radical polymerization initiator, for example, hydrogen peroxide or persulfate such as ammonium persulfate or potassium persulfate, hydroperoxides such as t-butyl hydroperoxide or cumene hydroperoxide, azo type initiator such as 2,2'-azobis(2-amidinopropane) dihydrogen chloride, or such water soluble initiator may be combined with, for example, a reducing substance such as sodium hydrogen sulfite or amines such as N,N,N',N'-tetramethylethylene diamine and used as a redox type initiator. The amount of the water soluble radical polymerization initiator used is from 0.01 to 10 wt% and, preferably, from 0.1 to 2 wt%.

Preferred embodiments of the invention are exemplified in the dependent claims.

### Best Mode for Practicing the Invention

The present invention will be explained more specifically with reference to examples.

Evaluation for the present invention will be measured by the following method.

### (1) Water Absorbing Rate

After sealing 0.2 g of a temperature sensitive water absorbing and discharging composition in a non-woven fabric bag, and immersing it in purified water controlled to a temperature of 10°C for 10 min, it was sufficiently drained and weighed. Separately, a non-woven fabric bag not containing the composition was treated in the same manner and it was used as a blank. A blank was subtracted from the resultant measured value, and a value converted into the weight per 1 g of the dry composition was defined as the water absorbing rate. As the value is larger, the water absorbing rate is greater.

### (2) Water Discharging Rate

After sealing 0.2 g of a temperature sensitive water absorbing and discharging composition in a non-woven fabric bag and immersing it in purified water controlled to a temperature of 10°C for 15 hours, sufficiently causing water to be absorbed, it was placed in purified water controlled to a temperature of 50°C, water was drained from the composition and a time till complete draining (time reaching a constant weight) was measured, and the time was defined as a water discharging rate. As the value is smaller, the discharging rate is greater.

### (3) Water Absorbing Amount

After sealing 0.2 g of the temperature sensitive water absorbing and discharging composition into a non-woven fabric bag, and immersing it in purified water controlled to an optional temperature for one day, it was sufficiently drained and then weighed. Separately, a non-woven fabric bag not containing the composition was treated in the same manner and used as a blank. The blank was subtracted from the obtained measured value and a value converted into weight per 1 g of the dry composition was defined as a water absorbing amount at each temperature. As the value is greater, it has higher water absorbability.

### Example 1

In a 500 ml separable flask, were added 17.82 g of N-isopropyl acrylamide, 1.48 g of an aqueous 40% aqueous solution of sodium acrylate, 8.88 g of diacetone acrylamide, 0.1 g of N,N-methylenebis acrylamide and 150 ml of purified water. Then, in a nitrogen atmosphere, 0.24 g of ammonium persulfate and 0.30 ml of N,N,N',N'-tetramethylethylene diamine were added at 10°C to start polymerization. After the copolymerization was over, it was warmed and a gel was taken out and dried in an electric drier at 100°C. The dried gel (resin) was pulverized and the water absorbing factor was measured. The resultant water absorbing factor is shown in Table 1.

### Example 2

The procedures were the same as those in Example 1 except for using 20.03 g of N,N-diethyl acrylamide instead of N-isopropyl acrylamide in Example 1 to obtain a resin. The water absorbing factor of the resultant resin is shown in Table 1.

### Comparative Example 1

A resin was obtained by the same procedures as those in Example 1 except for not using diacetone acrylamide and sodium acrylate and replacing them with 23.76 g of N-isopropyl acrylamide in Example 1. The water absorbing factor of the resultant resin is shown in Table 1.

### Comparative Example 2

A resin was obtained by the same procedures as those in Example 2 except for not using diacetone acrylamide and sodium acrylate and replacing them with 26.71 g of N,N-diethyl acrylamide in Example 2. The water absorbing factor of the resultant resin is shown in Table 1.

### Comparative Example 3

A resin was obtained by the same procedures as those in Example 1 except for not using diacetone acrylamide and replacing it with 23.76 g of N-isopropyl acrylamide in Example 1. The water absorbing factor of the resultant resin is shown in Table 1.

### Comparative Example 4

A resin was obtained by the same procedures as those in Example 1 except for not using diacetone acrylamide and replacing it with 4.52 g of methyl acrylate as a hydrophobic monomer in Example 1. The water absorbing factor of the resultant resin is shown in Table 1.

### Comparative Example 5

A resin was obtained by the same procedures as those in Example 1 except for not using diacetone acrylamide and replacing it with 6.73 g of n-butyl acrylate as a hydrophobic monomer in Example 1. The resultant resin was not uniform. Since the resin in this case was not uniform, measurement for the water absorbing factor was not conducted.

### Comparative Example 6

A resin was obtained by the same procedures as those in Example 1 except for not using diacetone acrylamide and replacing it with 4.47 g of methacrylamide as a water soluble monomer in Example 1. The water absorbing factor of the resultant resin is shown in Table 1.

### Example 3

A resin was obtained by the same procedures as those in Example 1 except for replacing sodium acrylate with 1.06 g of acrylic acid, ammonium sulfate and N,N,N',N'-tetramethylethylene diamine as the polymerization initiator with 0.027 ml of tert-butylhydroperoxide and 0.033 g of LONGARIT (WaKo Junyaku) respectively. The water absorbing factor of the resultant resin is shown in Table 1.

### Comparative Example 7

A resin was obtained by the same procedures as those in Example 1 except for not using diacetone acrylamide and replacing it with 23.76 g of N-isopropyl acrylamide in Example 3. The water absorbing factor of the resultant resin is shown in Table 1.

### Comparative Example 8

A resin was obtained by the same procedures as those in Example 3 except for not using diacetone acrylamide and replacing the amount of methyl acrylate as a hydrophobic monomer into 4.52 g in Example 3. The water absorbing factor of the resultant resin is shown in Table 1.

**Table 1**

| Gel used | Temperature (^{o}C) | | | | |
|---|---|---|---|---|---|
| | 10 | 20 | 30 | 40 | 50 |
| Example 1 | 247 | 224 | 57 | 12 | 3 |
| Example 2 | 186 | 128 | 23 | 7 | 3 |
| | | | | | |
| Comp. Example 1 | 41 | 36 | 25 | 3 | - |
| Comp. Example 2 | 29 | 22 | 5 | 4 | - |
| Comp. Example 3 | 251 | 247 | 240 | 226 | 226 |
| Comp. Example 4 | 266 | 260 | 250 | 199 | 52 |
| Comp. Example 6 | 230 | 226 | 220 | 207 | 193 |
| | | | | | |
| Example 3 | 142 | 134 | 9 | 2 | - |
| | | | | | |
| Comp. Example 7 | 150 | 141 | 130 | 15 | 2 |
| Comp. Example 8 | 142 | 125 | 100 | 13 | 3 |

As apparent from Table 1, the resins according to the present invention maintain higher water absorbability and temperature sensitive property as compared with other resins.

### Examples 4 - 8

Resins were obtained quite in the same procedures as those in Example 1 except for using combinations of the amounts of monomers described in Table 2. The water absorbing factors of the resultant resins are shown in Table 3.

**Table 2**

| Example | N-isopropyl acrylamide (g) | Diacetone acrylamide (g) |
|---|---|---|
| 4 | 16.63 | 10.66 |
| 5 | 19.01 | 7.11 |
| 6 | 20.20 | 5.33 |
| 7 | 21.39 | 3.55 |
| 8 | 22.58 | 1.78 |

**Table 3**

| Gel used | Temperature (^{o}C) | | | | |
|---|---|---|---|---|---|
| | 10 | 20 | 30 | 40 | 50 |
| Example 4 | 200 | 162 | 40 | 9 | 3 |
| Example 5 | 227 | 216 | 141 | 27 | 5 |
| Example 6 | 235 | 224 | 170 | 63 | 12 |
| Example 7 | 228 | 208 | 185 | 96 | 14 |
| Example 8 | 224 | 214 | 194 | 154 | 33 |

### Examples 9 - 12

Resins were obtained quite in the same procedures as those in Example 3 except for using combinations of the amounts of monomers described in Table 4 and changing the amount of acrylic acid to 0.47 g. The water absorbing factors of the resultant resins are shown in Table 5.

**Table 4**

| Example | N-isopropyl acrylamide (g) | Diacetone acrylamide (g) |
|---|---|---|
| 9 | 19.01 | 7.11 |
| 10 | 20.20 | 5.33 |
| 11 | 21.39 | 3.55 |
| 12 | 22.58 | 1.78 |

**Table 5**

| Gel used | Temperature (^{o}C) | | | | |
|---|---|---|---|---|---|
| | 15 | 20 | 25 | 30 | 35 |
| Example 9 | 68 | 60 | 3 | 2 | 1 |
| Example 10 | 67 | 57 | 24 | 2 | 1 |
| Example 11 | 76 | 72 | 55 | 12 | 2 |
| Example 12 | 85 | 84 | 77 | 62 | 9 |

As apparent from Tables 3 and 5, the temperature sensitive point can be changed by changing the amount of diacetone acrylamide introduced while maintaining high water absorbability.

### Example 13

300 ml of purified water was charged in a 500 ml separable flask and, under stirring, 20.44 g of N-isopropyl acrylamide, 4.97 g of diacetone acrylamide, 1.06 g of acrylic acidic, 0.14 g of N,N'-methylenebis acrylamide, 2.66 g of acid white clay (NIKKANITE S-200, manufactured by Nippon Kassei Hakudo Co.) were added, dissolved and dispersed. After cooling under a nitrogen gas stream till the liquid temperature was lowered to the 0°C, 0.099 g of LONGARIT and 0.081 ml of t-butyl hydroperoxide were added to conduct polymerization under heat insulating condition. After the reaction was over, a gel was taken out of the flask, dried at 100°C and then pulverized and classified, to obtain a composition (powder) with a grain size of 150 to 500 µm.

Water absorbing rate, water charging rate and water absorbing amount were measured for the resultant composition.

The results are shown in Table 1.

### Examples 14- 23

Compositions were obtained by using monomers, inorganic particles as described in Tables 6 and Table 7, and by the same procedures as those in Example 13. Same evaluation was also conducted to the resultant compositions and the results are shown in Table 6 and Table 7.
(1) Symbols in the tables are as below.

| Monomer | | Inorganic particles | |
|---|---|---|---|
| NIPAN | N-isopropyl acrylamide | J | Acidic white clay (Nippon Kassei Hakudo) |
| | | | |
| AAm | Acrylamide | K | kaolinite (Wako Junyaku) |
| DAAM | Diacetone acrylamide | D | Diatomaceous earth (Wako Junyaku) |
| | | | |
| AAc | Acrylic acid | | |
| MBAAm | N,N-methylene-bisacrylamide | | |

(2) Unit for the monomer amount (g)
Unit for the water absorbing amount at each temperature (g/g)

**Table 6**

| | Example | | | | | |
|---|---|---|---|---|---|---|
| | 13 | 14 | 15 | 16 | 17 | 18 |
| Amount of monomer | | | | | | |
| | | | | | | |
| NIPAN | | | 20.44 | | | |
| | | | | | | |
| AAm | | | | | | |
| | | | | | | |
| DAAm | | | 4.97 | | | |
| | | | | | | |
| AAc | | | 1.06 | | | |
| | | | | | | |
| MBAAm | | | 0.14 | | | |
| | | | | | | |

| Inorganic particle | | | | | | |
|---|---|---|---|---|---|---|
| | | | | | | |
| Kind | J | | | | | |
| | | | | | | |
| Amount (g) | 2.66 | 0.27 | 1.33 | 5.32 | 10.64 | 15.96 |
| | | | | | | |
| Water absorbing rate (g/g) | 50 | 12 | 40 | 47 | 45 | 30 |
| | | | | | | |
| Water discharging rate (min) | 40 | | | | | |
| | | | | | | |

| Water absorbing amount at each temperature | | | | | | |
|---|---|---|---|---|---|---|
| | | | | | | |
| 10°C | 120 | 122 | 115 | 122 | 130 | 116 |
| | | | | | | |
| 15°C | 115 | 118 | 112 | 120 | 125 | 110 |
| | | | | | | |
| 20°C | 115 | 115 | 110 | 118 | 120 | 105 |
| | | | | | | |
| 25°C | 110 | 110 | 106 | 115 | 118 | 105 |
| | | | | | | |
| 30°C | 7 | 6 | 5 | 8 | 6 | 5 |
| | | | | | | |
| 35°C | 5 | 5 | 5 | 5 | 5 | 5 |
| | | | | | | |
| 40°C | 5 | 5 | 5 | 5 | 5 | 5 |

**Table 7**

| | Example | | | | | |
|---|---|---|---|---|---|---|
| | 13 | 19 | 20 | 21 | 22 | 23 |
| Amount of monomer | | | | | | |
| | | | | | | |
| NIPAN | 20.44 | 22.58 | 20.44 | 20.44 | 20.44 | 22.58 |
| | | | | | | |
| AAm | | 0.75 | | | | 0.75 |
| | | | | | | |
| DAAm | 4.97 | | 4.97 | 4.97 | 4.97 | |
| | | | | | | |
| AAc | 1.06 | 1.82 | 1.06 | 1.06 | 1.06 | 1.82 |
| | | | | | | |
| MBAAm | 0.14 | 0.14 | 0.14 | 0.14 | 0.14 | 0.14 |
| | | | | | | |

| Inorganic particle | | | | | | |
|---|---|---|---|---|---|---|
| | | | | | | |
| Kind | J | J | K | D | | |
| | | | | | | |
| Amount (g) | 2.66 | 2.44 | 2.66 | 2.66 | | |
| | | | | | | |
| Water absorbing rate (g/g) | 50 | 50 | 45 | 43 | 15 | 12 |
| | | | | | | |
| Water discharging rate (min) | 40 | | | | 85 | |
| | | | | | | |

| Water absorbing amount at each temperature | | | | | | |
|---|---|---|---|---|---|---|
| | | | | | | |
| 10°C | 120 | 125 | 118 | 135 | 122 | 118 |
| | | | | | | |
| 15°C | 115 | 120 | 117 | 132 | 118 | 110 |
| | | | | | | |
| 20°C | 115 | 115 | 110 | 128 | 108 | 105 |
| | | | | | | |
| 25°C | 110 | 113 | 60 | 110 | 105 | 103 |
| | | | | | | |
| 30°C | 7 | 110 | 7 | 10 | 8 | 100 |
| | | | | | | |
| 35°C | 5 | 55 | 5 | 8 | 3 | 50 |
| | | | | | | |
| 40°C | 5 | 5 | 5 | 5 | 3 | 5 |

As described above, the feature of the present invention resides in introducing diacetone acrylamide as a water soluble monomer for controlling the temperature sensing point to a lower temperature region optionally in a state of maintaining a high water absorbing factor.

A method of introducing a hydrophobic monomer for controlling the temperature sensing point to a low temperature region has been known. However, since the hydrophobic monomer has a low water solubility, it involves drawbacks, for example, in that polymerization in an aqueous solution is impossible or, even if possible, optional control of the temperature sensing point is impossible since the amount of introduction is limited, and, further, the concentration of the monomer upon polymerization has to be reduced an extremely low concentration. For instance, methyl acrylate known as the hydrophobic monomer has a water solubility of about 6%, and inhomogeneous polymerizate is formed at a concentration higher than that. Further, assuming the water absorbing factor at 10°C as 100 and if the temperature at which the water absorbing factor is reduced to 50 is defined as the temperature sensing point, the temperature sensing point of the polymer is lowered only by 1.7°C in a case of introducing 10 mol% of methyl acrylate as described in Comparative Example 8, whereas the temperature sensing point is lowered extremely as much as by 7°C in the case of diacetone acrylamide as described in Examples 9 - 12. Since diacetone acrylamide used in the present invention is a water soluble monomer and dissolves in water at an optional ratio, a homogeneous resin can be produced easily and the content can be varied optionally. Accordingly, it has an advantage capable of easily setting the temperature sensing point in accordance with on the working temperature while maintaining a high water absorbing factor as it is

### Industrial Applicability

As has been explained above, since the temperature sensitive water absorbing and discharging composition according to the present invention can control the temperature sensing point and high water discharging and absorbing property above and below the temperature sensing point, it can be applied to a wide variety of range, for example, sanitary materials such as paper diaper, soil improvers in agricultural and horticultural fields for plants and vegetables, such as soil water feeding agents or soil water retaining agents, water proof sealing materials, sandbags, temperature sensors, chemical valves, solid-liquid separation and concentrating materials.

## Claims

1. A temperature sensitive water absorbing and discharging polymer composition formed by copolymerizing an aqueous solution containing N-isopropyl acrylamide and/or N,N-diethyl acrylamide, acrylic acid and/or alkali metal salt of acrylic acid, and diacetone acrylamide and/or acrylamide in the presence of a crosslinking agent in an aqueous solution.

2. A temperature sensitive water absorbing and discharging polymer composition formed by copolymerizing an aqueous solution containing N-isopropyl acrylamide, acrylic acid and diacetone acrylamide in the presence of a crosslinking agent in an aqueous solution.

3. A temperature sensitive water absorbing and discharging polymer composition formed by copolymerizing an aqueous solution containing N-isopropyl acrylamide, acrylic acid and acrylamide in the presence of a cross-linking agent in an aqueous solution.

4. A temperature sensitive water absorbing and discharging polymer composition formed by copolymerizing an aqueous solution containing N-isopropyl acrylamide and/or N,N-diethyl acrylamide, acrylic acid and/or alkali metal salt of acrylic acid, and diacetone acrylamide and/or acrylamide in the presence of inorganic particles and a crosslinking agent in an aqueous solution.

5. A temperature sensitive water absorbing and discharging polymer composition formed by copolymerizing an aqueous solution containing N-isopropyl acrylamide, acrylic acid and diacetone acrylamide in the presence of inorganic particles and a crosslinking agent in an aqueous solution.

6. A temperature sensitive water absorbing and discharging polymer composition formed by copolymerizing an aqueous solution containing N-isopropyl acrylamide, acrylic acid and acrylamide in the presence of inorganic particles and a crosslinking agent in an aqueous solution.

7. A temperature sensitive water absorbing and discharging polymer composition as defined in claim 4, 5 or 6, wherein the inorganic particles are one or more of materials selected from acidic white clay, diatomaceous earth and kaolinite.

8. A process for producing a temperature sensitive water absorbing and discharging polymer composition, which comprises copolymerizing in an aqueous solution N-isopropyl acrylamide and/or N,N-diethyl acrylamide, acrylic acid and/or alkali metal salt of acrylic acid and diacetone acrylamide in the presence of a crosslinking agent, wherein inorganic particles are present.

9. Utilization of a temperature sensitive water absorbing and discharging polymer composition formed by copolymerizing an aqueous solution containing N-isopropyl acrylamide and/or N,N-diethyl acrylamide, acrylic acid and/or alkali metal salt of acrylic acid, and diacetone acrylamide and/or acrylamide in the presence of a crosslinking agent in an aqueous solution, to sanitary materials such as paper diapers, soil improvers in agricultural and horticultural fields for plants and vegetables such as soil water feeding agents or soil water retaining agents, water proof sealing materials, sandbags, temperature sensors, chemical valves, solid-liquid separation and concentrating materials.

## Patentansprüche

1. Temperaturempfindliche, wasserabsorbierende und freigebende Polymerzusammensetzung, gebildet durch Copolymerisieren einer wäßrigen Lösung, die N-Isopropylamid und/oder N,N-Diethylacrylamid, Acrylsäure und/oder Alkalimetallsalz von Acrylsäure und Diacetonacrylamid und/oder Acrylamid enthält, in Gegenwart eines Vernetzungsmittels in einer wäßrigen Lösung.

2. Temperaturempfindliche, wasserabsorbierende und freigebende Polymerzusammensetzung, gebildet durch Copolymerisieren einer wäßrigen Lösung, die N-Isopropylacrylamid, Acrylsäure und Diacetonacrylamid enthält, in Gegenwart eines Vernetzungsmittels in einer wäßrigen Lösung.

3. Temperaturempfindliche, wasserabsorbierende und freigebende Polymerzusammensetzung, gebildet durch Copolymerisieren einer wäßrigen Lösung, die N-Isopropylacrylamid, Acrylsäure und Acrylamid enthält, in Gegenwart eines Vernetzungsmittels in einer wäßrigen Lösung.

4. Temperaturempfindliche, wasserabsorbierende und freigebende Polymerzusammensetzung, gebildet durch Copolymerisieren einer wäßrigen Lösung, die N-Isopropylacrylamid und/oder N,N-Diethylacrylamid, Acrylsäure und/oder Alkalimetallsalz von Acrylsäure und Diacetonacrylamid und/oder Acrylamid enthält, in Gegenwart von anorganischen Partikeln und einem Vernetzungsmittel in einer wäßrigen Lösung.

5. Temperaturempfindliche, wasserabsorbierende und freigebende Polymerzusammensetzung, gebildet durch Copolymerisieren einer wäßrigen Lösung, die N-Isopropylacrylamid, Acrylsäure und Diacetonacrylamid enthält, in Gegenwart von anorganischen Partikeln und einem Vernetzungsmittel in einer wäßrigen Lösung.

6. Temperaturempfindliche, wasserabsorbierende und freigebende Polymerzusammensetzung, gebildet durch Copolymerisieren einer wäßrigen Lösung, die N-Isopropylacrylamid, Acrylsäure und Acrylamid enthält, in Gegenwart von anorganischen Partikeln und einem Vernetzungsmittel in einer wäßrigen Lösung.

7. Temperaturempfindliche, wasserabsorbierende und freigebende Polymerzusammensetzung nach Anspruch 4, 5 oder 6, worin die anorganischen Partikel ein oder mehrere Materialien sind, ausgewählt aus saurem weißen Ton, Diatomeenerde und Kaolinit.

8. Verfahren zur Herstellung einer temperaturempfindlichen, wasserabsorbierenden und freigebenden Polymerzusammensetzung, welches das Copolymerisieren in einer wäßrigen Lösung von N-Isopropylacrylamid und/oder N,N-Diethylacrylamid, Acrylsäure und/oder Alkalimetallsalz von Acrylsäure und Diacetonacrylamid in Gegenwart eines Vernetzungsmittels, worin anorganische Partikel vorliegen, umfaßt.

9. Verwendung einer temperaturempfindlichen, wasserabsorbierenden und freigebenden Polymerzusammensetzung, gebildet durch Polymerisieren einer wäßrigen Lösung, die N-Isopropylacrylamid und/oder Alkalimetallsalz von Acrylsäure und Diacetonacrylamid und/oder Acrylamid enthält, in Gegenwart eines Vernetzungsmittels in einer wäßrigen Lösung, für Sanitärmaterialien wie Papier, Windeln, Bodenverbesserer in den Bereichen Landwirtschaft und Gartenbau für Pflanzen und Gemüse, wie Bodenwasser- Zuführstoffe oder Bodenwasser-Zurückhaltstoffe, wasserfeste Versiegelungsmaterialien, Sandsäcke, Temperatursensoren, chemische Ventile, Fest-Flüssigtrennung und Konzentrierungsmaterialien.

## Revendications

1. Composition de polymère absorbant et libérant de l'eau, sensible à la température, formée par copolymérisation d'une solution aqueuse contenant du N-isopropylacrylamide et/ou du N,N-diéthylacrylamide, de l'acide acrylique et/ou un sel de métal alcalin d'acide acrylique, et du diacétoneacrylamide et/ou de l'acrylamide en présence d'un agent de réticulation en solution aqueuse.

2. Composition de polymère absorbant et libérant de l'eau, sensible à la température, formée par copolymérisation d'une solution aqueuse contenant du N-isopropylacrylamide, de l'acide acrylique, et du diacétoneacrylamide en présence d'un agent de réticulation en solution aqueuse.

3. Composition de polymère absorbant et libérant de l'eau, sensible à la température, formée par copolymérisation d'une solution aqueuse contenant du N-isopropylacrylamide, de l'acide acrylique, et de l'acrylamide en présence d'un agent de réticulation en solution aqueuse.

4. Composition de polymère absorbant et libérant de l'eau, sensible à la température, formée par copolymérisation d'une solution aqueuse contenant du N-isopropylacrylamide et/ou du N,N-diéthylacrylamide, de l'acide acrylique et/ou un sel de métal alcalin d'acide acrylique, et du diacétoneacrylamide et/ou de l'acrylamide en présence de particules inorganiques et d'un agent de réticulation en solution aqueuse.

5. Composition de polymère absorbant et libérant de l'eau, sensible à la température, formée par copolymérisation d'une solution aqueuse contenant du N-isopropylacrylamide, de l'acide acrylique, et du diacétoneacrylamide en présence de particules inorganiques et d'un agent de réticulation en solution aqueuse.

6. Composition de polymère absorbant et libérant de l'eau, sensible à la température, formée par copolymérisation d'une solution aqueuse contenant du N-isopropylacrylamide, de l'acide acrylique, et de l'acrylamide en présence de particules inorganiques et d'un agent de réticulation en solution aqueuse.

7. Composition de polymère absorbant et libérant de l'eau, sensible à la température, selon la revendication 4, 5 ou 6, dans laquelle les particules inorganiques sont un ou plusieurs matériaux choisis parmi l'argile blanche acide, la terre de diatomées et la kaolinite.

8. Procédé de production d'une composition de polymère absorbant et libérant de l'eau, sensible à la température, qui comprend la copolymérisation, dans une solution aqueuse de N-isopropylacrylamide et/ou de N,N-diéthylacrylamide, d'acide acrylique et/ou d'un sel de métal alcalin d'acide acrylique et de diacétoneacrylamide en présence d'un agent de réticulation, dans lequel des particules inorganiques sont présentes.

9. Utilisation d'une composition de polymère absorbant et libérant de l'eau, sensible à la température, formée par copolymérisation d'une solution aqueuse contenant du N-isopropylacrylamide et/ou du N,N-diéthylacrylamide, de l'acide acrylique et/ou un sel de métal alcalin d'acide acrylique, et du diacétoneacrylamide et/ou de l'acrylamide en présence d'un agent de réticulation en solution aqueuse, pour des matériaux hygiéniques comme des couches en papier, des améliorants du sol dans les domaines de l'agriculture et de l'horticulture pour des plantes et des légumes comme des agents apportant de l'eau au sol ou des agents retenant l'eau dans le sol, des matériaux étanches à l'eau, des sacs de sable, des palpeurs pyrométriques, des valves des valves chimiques, des matériaux de concentration et de séparation solide-liquide.
